(19) Europäisches Patentamt
European Patent Office
Office européen des brevets

(11) EP 0 667 331 B1

(12) FASCICULE DE BREVET EUROPEEN

(45) Date de publication et mention de la délivrance du brevet: **08.12.1999 Bulletin 1999/49**

(51) Int Cl.[6]: **C07C 45/38**, C07C 47/565, C07C 47/575

(21) Numéro de dépôt: **95400240.8**

(22) Date de dépôt: **06.02.1995**

# (54) Procédé de préparation d'hydroxybenzaldehydes

Verfahren zur Herstellung von Hydroxybenzaldehyden

Process for the preparation of hydroxybenzaldehydes

(84) Etats contractants désignés:
**AT BE CH DE DK ES FR GB IE IT LI NL PT SE**

(30) Priorité: **11.02.1994 FR 9401562**

(43) Date de publication de la demande:
**16.08.1995 Bulletin 1995/33**

(73) Titulaire: **RHODIA CHIMIE**
**92408 Courbevoie Cédex (FR)**

(72) Inventeur: **Lefranc, Hélène**
**F-69630 Chaponost (FR)**

(74) Mandataire: **Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
**DE-B- 1 188 069**
**FR-A- 550 293**
**FR-A- 2 305 420**
**US-A- 4 351 962**

- **CHEMICAL ABSTRACTS, vol. 86, no. 11, 14 Mars 1977, Columbus, Ohio, US; abstract no. 72121p, CERVENY, LIBOR ET AL. 'Optimizing a two-stage synthesis of salicylaldehyde from phenol.' page 568 ;colonne 1 ; & CHEM. TECH., vol.28, no.9, 1976, LEIPZIG pages 557 - 559**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 667 331 B1

Printed by Jouve, 75001 PARIS (FR)

## Description

La présente invention a pour objet un procédé d'oxydation d'alcools hydroxybenzyliques en vue de préparer les hydroxybenzaldéhydes correspondants.

L'invention a trait plus particulièrement à la préparation d'aldéhyde salicylique, à partir de l'alcool ortho-hydroxybenzylique communément appelé saligénol.

Divers procédés ont été proposés pour réaliser cette oxydation.

Il est connu, en particulier, selon FR-A 2 305 420, de réaliser l'oxydation de l'alcool ortho-hydroxybenzylique, en phase liquide, à l'aide d'oxygène moléculaire ou un gaz en contenant, en opérant en milieu aqueux renfermant un agent alcalin, en présence d'un catalyseur à base de palladium ou de platine, la caractéristique de ce procédé étant de conduire l'oxydation, en présence d'un co-catalyseur à base d'un dérivé du bismuth.

Les catalyseurs à base de platine qui donnent de meilleurs rendements réactionnels que les catalyseurs à base de palladium, sont accrus, selon l'invention grâce à la présence de bismuth. Ainsi, les rendements en aldéhyde salicylique annoncés sont de 77,6 % en l'absence de bismuth, et de 92,8 % en présence de bismuth.

L'objectif de la présente invention est de disposer d'un procédé permettant d'améliorer encore le rendement de la réaction d'oxydation.

Il a maintenant été trouvé et c'est ce qui fait l'objet de la présente invention, un procédé de préparation d'un hydroxybenzaldéhyde par oxydation de l'alcool hydroxybenzylique correspondant, en phase liquide, à l'aide d'oxygène moléculaire ou un gaz en contenant, en opérant en milieu aqueux renfermant un agent alcalin, en présence d'un catalyseur à base de platine, caractérisé par le fait que l'oxydation est conduite, en présence d'un dérivé du bore et d'un dérivé du bismuth.

Il a été trouvé que, dans le cas d'un catalyseur à base de platine, la présence d'un dérivé du bore, conjointement avec le dérivé du bismuth permettait d'obtenir lors de l'oxydation du saligénol, des rendements encore plus élevés en aldéhyde salicylique pouvant atteindre 97 %.

La présence de bore permet de limiter l'oxydation au stade de l'aldéhyde, sans formation d'acide.

Le procédé de l'invention s'applique à tout alcool hydroxybenzylique c'est-à-dire à tout composé aromatique porteur d'au moins un groupe -OH et d'un groupe $-CH_2OH$.

Par "composé aromatique", on entend la notion classique d'aromaticité telle que définie dans la littérature, notamment par Jerry MARCH, Advanced Organic Chemistry, $3^{ème}$ édition, John Wiley and Sons, 1985, p.37 et suivantes.

L'invention est particulièrement adaptée pour oxyder les alcools hydroxybenzyliques répondant à la formule (I) suivante :

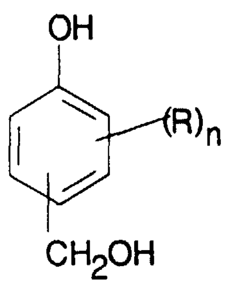

(I)

dans ladite formule, le radical $-CH_2OH$ est en position ortho, méta ou para du groupe hydroxyle et le noyau benzénique peut être éventuellement substitué par un ou plusieurs substituants R, identiques ou différents et n est un nombre inférieur ou égal à 3.

Dans l'exposé qui suit de la présente invention, le radical de formule

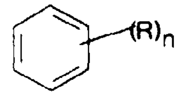

est symbolisé par "Ar".

N'importe quel substituant peut être présent sur le noyau benzénique dans la mesure où il n'interfère pas au niveau du produit désiré. Comme exemples de substituants R, on peut citer, notamment les atomes d'halogène, de

préférence, fluor, chlore ou brome et les radicaux alkyle ou alkoxy ayant de préférence de 1 à 12 atomes de carbone, de préférence de 1 à 4 atomes de carbone.

Comme exemples d'alcools hydroxybenzyliques préférés, on peut mentionner :

- l'alcool hydroxy-2 benzylique,
- l'alcool hydroxy-2 méthyl-4 benzylique,
- l'alcool hydroxy-2 méthyl-6 benzylique,
- l'alcool hydroxy-2 éthoxy-6 benzylique,
- l'alcool hydroxy-2 chloro-6 benzylique.

Le procédé de l'invention est tout particulièrement intéressant pour la préparation industrielle de l'aldéhyde salicylique par oxydation du saligénol.

En ce qui concerne les dérivés du bore, on met en oeuvre de préférence les acides boriques tels que l'acide orthoborique couramment appelé acide borique (ou son précurseur $B_2O_3$), l'acide métaborique, l'acide pyro- ou tétraborique.

On peut également faire appel aux borates métalliques, notamment de métaux alcalins, alcalino-terreux ou d'ammonium sous forme anhydre ou hydratée, en particulier aux tiers borates, hémiborates, monoborates, diborates, triborates, tétraborates, pentaborates de métaux, de préférence alcalins ou d'ammonium.

On peut également avoir recours à un sel double contenant du bore, notamment les fluoborates métalliques, par exemple, le fluoborate de potassium.

Comme exemples de composés du bore convenant à l'invention, on peut citer :

- l'orthoborate de sodium,
- l'orthoborate de potassium,
- le monohydrogénoorthoborate de sodium,
- le monohydrogénoorthoborate de potassium,
- le dihydrogénoorthoborate de sodium,
- le dihydrogénoorthoborate de potassium,
- l'acide orthoborique ou son précurseur, l'anhydride borique,
- le métaborate de sodium,
- le métaborate tétrahydraté de sodium,
- le tétraborate de sodium,
- le tétraborate décahydraté de sodium ou borax,
- le tétraborate pentahydraté de sodium,
- le métaborate de potassium,
- le pentaborate tétrahydraté de potassium,
- le tétraborate octahydraté de potassium,
- le pentaborate tétrahydraté d'ammonium,
- le tétraborate tétrahydraté d'ammonium.

D'une manière préférentielle, on fait appel à l'acide borique ou à l'anhydride borique.

Pour ce qui est de la quantité de dérivé du bore à mettre en oeuvre, elle est déterminée de telle sorte que le rapport entre le nombre de moles de dérivé du bore et le nombre de moles d'alcool hydroxybenzylique varie entre entre 0,1 et 3,0, de préférence, entre 0,9 et 1,1.

Comme co-catalyseur, on fait appel en général à un dérivé minéral ou organique du bismuth dans lequel l'atome de bismuth se trouve à un degré d'oxydation supérieur à zéro, par exemple égal à 2, 3, 4 ou 5. Le reste associé au bismuth n'est pas critique dès l'instant qu'il satisfait à cette condition. Le co-catalyseur peut être soluble ou insoluble dans le milieu réactionnel.

Des composés illustratifs de co-catalyseurs qui peuvent être utilisés dans le procédé selon la présente invention sont : les oxydes de bismuth ; les hydroxydes de bismuth ; les sels d'hydracides minéraux tels que : chlorure, bromure, iodure, sulfure, séléniure, tellure de bismuth ; les sels d'oxyacides minéraux tels que : sulfite, sulfate, nitrite, nitrate, phosphite, phosphate, pyrophosphate, carbonate, perchlorate, antimoniate, arséniate, sélénite, séléniate de bismuth ; les sels d'oxyacides dérivés de métaux de transition tels que : vanadate, niobate, tantalate, chromate, molybdate, tungstate, permanganate de bismuth.

D'autres composés appropriés sont également des sels d'acides organiques aliphatiques ou aromatiques tels que : acétate, propionate, benzoate, salicylate, oxalate, tartrate, lactate, citrate de bismuth ; des phénates tels que : gallate et pyrogallate de bismuth. Ces sels et phénates peuvent être aussi des sels de bismuthyle.

Comme autres composés minéraux ou organiques, on peut utiliser des combinaisons binaires du bismuth

avec des éléments tels que phosphore et arsenic ; des hétéropolyacides contenant du bismuth ainsi que leurs sels ; conviennent également les bismuthines aliphatiques et aromatiques.

A titre d'exemples spécifiques, on peut citer :

- comme oxydes : BiO; ; $Bi_2O_3$: $Bi_2O_4$ ; $Bi_2O_5$.
- comme hydroxydes : $Bi(OH)_3$,
- comme sels d'hydracides minéraux : le chlorure de bismuth $BiCl_3$ ; le bromure de bismuth $BiBr_3$ , l'iodure de bismuth $BiI_3$ ; le sulfure de bismuth $Bi_2S_3$ ; le séléniure de bismuth $Bi_2Se_3$ : le tellure de bismuth $Bi_2Te_3$,
- comme sels d'oxyacides minéraux : le sulfite basique de bismuth $Bi_2(SO_3)_3$, $Bi_2O_3$, $5H_2O$ ; le sulfate neutre de bismuth $Bi_2(SO_4)_3$ ; le sulfate de bismuthyle $(BiO)HSO_4$ ; le nitrite de bismuthyle $(BiO)NO_2$, $0{,}5H_2O$ ; le nitrate neutre de bismuth $Bi(NO_3)_3$, $5H_2O$ ; le nitrate double de bismuth et de magnésium $2Bi(N0_3)_3$, $3Mg(NO_3)_2$, $24H_2O$ ; le nitrate de bismuthyle $(BiO)NO_3$ ; le phosphite de bismuth $Bi_2(PO_3H)_3$, $3H_2O$ ; le phosphate neutre de bismuth $BiPO_4$ ; le pyrophosphate de bismuth $Bi_4(P_2O_7)_3$ ; le carbonate de bismuthyle $(BiO)_2CO_3$, $0{,}5H_2O$ ; le perchlorate neutre de bismuth $Bi(ClO_4)_3$, $5H_2O$ ; le perchlorate de bismuthyle $(BiO)ClO_4$ ; l'antimoniate de bismuth $BiSbO_4$ ; l'arséniate neutre de bismuth $Bi(AsO_4)_3$ ; l'arséniate de bismuthyle $(BiO)AsO_4$, $5H_2O$ ; le sélénite de bismuth $Bi_2(SeO_3)_3$.
- comme sels d'oxyacides dérivés de métaux de transition : le vanadate de bismuth $BiVO_4$ ; le niobate de bismuth BiNbO : le tantalate de bismuth $BiTaO_4$ ; le chromate neutre de bismuth $Bi_2(CrO_4)$ ; le dichromate de bismuthyle $(BiO)_2)_2Cr_2O_7$ ; le chromate acide de bismuthyle $H(BiO)CrO_4$ ; le chromate double de bismuthyle et de potassium $K(BiO)CrO_{10}$ ; le molybdate de bismuth $Bi_2(MoO_4)_3$ ; le tungstate de bismuth $Bi_2(WO_4)_3$ ; le molybdate double de bismuth et de sodium $NaBi(MoO_4)_2$ ; le permanganate basique de bismuth $Bi_2O_2(OH)MnO_4$.
- comme sels d'acides organiques aliphatiques ou aromatiques : l'acétate de bismuth $Bi(C_2H_3O_2)_3$ ; le propionate de bismuthyle $(BiO)C_3H_5O_2$ ; le benzoate basique de bismuth $C_6H_5CO_2Bi(OH)_2$ ; le salicylate de bismuthyle $C_6H_4CO_2(BiO)(OH)$ ; l'oxalate de bismuth $(C_2O_4)_3Bi_2$ ; le tartrate de bismuth $Bi_2(C_4H_4O_6)_3$, $6H_2O$ ; le lactate de bismuth $(C_6H_9O_5)OBi$, $7H_2O$ ; le citrate de bismuth $C_6H_5O_7Bi$.
- comme phénates : le gallate basique de bismuth $C_7H_7O_7Bi$ ; le pyrogallate basique de bismuth $C_6H_3(OH)_2(OBi)(OH)$.

Comme autres composés minéraux ou organiques conviennent également : le phosphure de bismuth BiP ; l'arséniure de bismuth $Bi_3As_4$ ; le bismuthate de sodium $NaBiO_3$ ; les acides bismuth-thiocyaniques $H_2[Bi(BNS)_5]$, $H_3[Bi(CNS)_6]$ et leurs sels de sodium et potassium ; la triméthylbismuthine $Bi(CH_3)_3$, la triphénylbismuthine $Bi(C_6H_5)_3$.

Les dérivés du bismuth qui sont utilisés de préférence pour conduire le procédé selon l'invention sont : les oxydes de bismuth ; les hydroxydes de bismuth ; les sels de bismuth ou de bismuthyle d'hydracides minéraux ; les sels de bismuth ou de bismuthyle d'oxyacides minéraux ; les sels de bismuth ou de bismuthyle d'acides organiques aliphatiques ou aromatiques ; et les phénates de bismuth ou de bismuthyle.

Un groupe de co-catalyseurs qui conviennent particulièrement bien à la réalisation de l'invention est constitué par : les oxydes de bismuth $Bi_2O_3$ et $Bi_2O_4$ ; l'hydroxyde de bismuth $Bi(OH)_3$ ; le sulfate neutre de bismuth $Bi_2(SO_4)_3$ ; le chlorure de bismuth $BiCl_3$ ; le bromure de bismuth $BiBr_3$ ; l'iodure de bismuth $BiI_3$ ; le nitrate neutre de bismuth $Bi(NO_3)_3$, $5H_2O$ ; le nitrate de bismuthyle $BiO(NO_3)$ ; le carbonate de bismuthyle $(BiO)_2CO_3$, $O{,}5H_2O$ ; l'acétate de bismuth $Bi(C_2H_3O_2)_3$ ; le salicylate de bismuthyle $C_6H_4CO_2(BiO)(OH)$.

La quantité de co-catalyseur utilisée, exprimée par la quantité de bismuth métallique contenue dans le co-catalyseur par rapport au poids du platine engagé, peut varier dans de larges limites. Par exemple, cette quantité peut être aussi petite que 0,1 % et peut atteindre le poids de platine engagé et même le dépasser sans inconvénient.

Plus particulièrement, cette quantité est choisie de manière qu'elle apporte dans le milieu d'oxydation de 10 à 900 ppm en poids de bismuth métallique par rapport à l'alcool hydroxybenzylique. A cet égard, des quantités supérieures de co-catalyseur de l'ordre de 900 à 1500 ppm peuvent naturellement être utilisées, mais sans avantage supplémentaire important.

En ce qui concerne le platine utilisé conjointement pour catalyser la réaction, il peut avoir diverses formes comme par exemple : le noir de platine, l'oxyde de platine, ou le métal noble lui-même déposé sur des supports divers tels que le noir de carbone, le carbonate de calcium, les alumines et silices activées ou des matériaux équivalents. Des masses catalytiques à base de noir de carbone conviennent particulièrement.

La quantité de ce catalyseur à mettre en oeuvre, exprimée en poids de platine métallique par rapport à celui de l'alcool à oxyder, peut varier de 0,01 à 4 % et, de préférence, de 0,04 à 2 %.

La concentration de l'alcool à oxyder dans la solution aqueuse d'agent alcalin doit de préférence être telle que l'on évite toute précipitation et conserve une solution homogène.

La concentration pondérale de l'alcool dans le milieu aqueux est habituellement comprise entre 1 % et 60 %, de préférence entre 2 % et 30 %.

Selon le procédé de l'invention, l'oxydation est conduite dans un milieu aqueux contenant en solution un agent

alcalin. A cet égard, on fait appel en général, comme agent alcalin, à l'hydroxyde de sodium ou de potassium. La proportion de base minérale à utiliser peut être comprise entre 0,5 et 3 moles de base minérale par mole d'alcool à oxyder.

Pratiquement une manière d'exécuter le procédé consiste à mettre en contact avec de l'oxygène moléculaire ou un gaz en contenant la solution aqueuse renfermant l'alcool à oxyder, l'agent alcalin, le catalyseur à base de platine, le co-catalyseur à base de dérivé du bismuth et le dérivé du bore, selon les proportions indiquées ci-dessus. On opère à pression atmosphérique, mais on peut aussi le cas échéant opérer sous pression. Le mélange est ensuite agité à la température désirée jusqu'à consommation d'une quantité d'oxygène correspondant à celle nécessaire pour transformer l'alcool en aldéhyde. On contrôle donc la marche de la réaction, par la mesure de la quantité d'oxygène absorbée.

La température réactionnelle à adopter varie selon la stabilité thermique des produits à préparer.

D'une façon générale, la réaction est conduite dans une gamme de température allant de 10°C à 100°C, de préférence allant de 20°C à 60°C.

Puis, après refroidissement s'il y a lieu, on sépare la masse catalytique de la masse réactionnelle, par exemple par filtration et on acidifie le liquide résultant par addition d'un acide protonique d'origine minérale, de préférence l'acide sulfurique jusqu'à obtention d'un pH inférieur ou égal à 6. Il suffit ensuite d'isoler l'hydroxybenzaldéhyde recherché, par exemple par extraction à l'aide d'un solvant convenable (par exemple le toluène) ou par entraînement à la vapeur d'eau, et de le purifier selon les procédés connus.

Conformément au procédé de l'invention, le dérivé du bore peut être introduit lors de l'oxydation, dans le cas où l'alcool hydroxybenzylique, plus particulièrement le saligénol, est préparé selon les techniques classiques décrites dans la littérature, par exemple, par condensation du phénol, avec le formaldéhyde en présence d'acétate de zinc ou formiate de calcium (GB 774 696).

Une autre variante de l'invention consiste à introduire le dérivé du bore, lors de la préparation de l'alcool hydroxybenzylique effectuée selon un enchaînement spécifique tel que décrit notamment dans FR-A 1 328 945 et FR-A 2 430 928.

Ainsi, dans une première étape, on effectue la préparation d'un ester borique de phénol par réaction d'un phénol et de l'acide borique (ou l'anhydride borique), puis on fait réagir l'ester borique de phénol obtenu avec le formaldéhyde ou un générateur de formaldéhyde, par exemple, le trioxane.

Les esters boriques obtenus dénommés de manière simplifiée "borates d'aryle", sont des mélanges complexes formés :

- de métaborates de phénols de formule (II) :

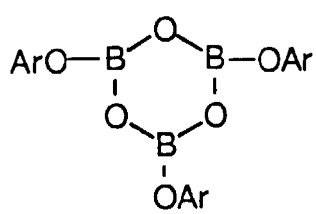

(II)

- de pyroborates de phénols de formule (III) :

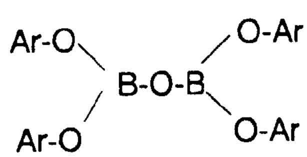

(III)

- d'orthoborates de phénols de formule (IV) :

$$(Ar\text{-}O\text{-})_3\text{-}B \qquad (IV)$$

- de borates acides de phénols de formule (V) :

$$(Ar\text{-}O\text{-})_2\text{-}B\text{-}OH \qquad (V)$$

(Ar ayant la signification donnée précédemment).

Lesdits mélanges contiennent éventuellement le phénol de départ en excès.
La proportion de chacun de ces dérivés de l'acide borique dans le mélange d'estérification varie selon le rapport molaire phénol/acide borique engagé. Ledit rapport est généralement compris entre 0,8 et 3,0, de préférence entre 1,0 et 1,5.
Ainsi, pour un rapport molaire phénol/acide borique compris entre 1,0 et 1,5, le mélange est principalement formé de métaborates de formule (II) ; et pour des rapports compris entre 1,5 et 3,0, il est formé essentiellement de pyroborates de phénols de formule (III) et de borates acides de phénols de formule (V) ; et pour un rapport égal ou voisin de 3,0, les orthoborates de formule (IV) sont pratiquement les seuls composants du mélange.
La préparation des borates d'aryle est réalisée conformément aux procédés connus, par réaction d'un phénol et de l'acide borique.
Le phénol répond de préférence à la formule :

$$Ar\text{-}OH \qquad (VI)$$

**[0051]** - Ar ayant la signification donnée précédemment.
Comme exemples de phénols de formule (VI), on peut citer : le phénol, les crésols, le xylénol-2,3, le xylénol-2,4, le xylénol-2,5, le xylénol-3,4, les monométhylphénols, les monoéthylphénols, les monopropylphénols, les monobutylphénols, les monométhyl-, les monoéthyl-, les monopropyl-, les monobutyléthers de pyrocatéchine, d'hydroquinone ou de résorcinol, les monochlorophénols, le 2,3-dichlorophénol, le 2,4-dichlorophénol, le 2,5-dichlorophénol, le 3,4-dichlorophénol, le 3,5-dichlorophénol, le 2,4,5-trichlorophénol, le 2,3,5-trichlorophénol, le 2,3-diméthoxyphénol, le 3,5-diméthoxyphénol.
La préparation du borate d'aryle par réaction du phénol et de l'acide borique est conduite, dans un solvant formant un azéotrope avec l'eau de la réaction d'estérification. Cette dernière est éliminée au fur et à mesure de sa formation par distillation azéotropique. Comme solvants convenant à la préparation des borates d'aryle, on peut citer, les hydrocarbures aromatiques tels que le benzène, le toluène, le xylène. Tout autre solvant inerte permettant la distillation azéotropique de l'eau peut être utilisé.
La réaction de condensation est conduite en milieu anhydre. On peut utiliser le solvant qui a servi à la préparation du borate d'aryle. Dans ces conditions, le borate n'est pas isolé du milieu après estérification et il est mis en réaction directement avec le formaldéhyde.
La quantité de formaldéhyde mise en oeuvre est de préférence égale à 1 mole par mole d'acide borique. Elle peut s'écarter sans inconvénient de cette valeur et peut être comprise entre 0,9 et 1,1.
Lorsque l'on utilise un générateur de formaldéhyde, la quantité de formaldéhyde est calculée pour que la quantité de formaldéhyde disponible à la réaction se situe dans l'intervalle précité.
La température de la condensation du formaldéhyde ou de son générateur et du phénol choisi, peut être comprise entre 20°C et 120°C, et de préférence entre 40°C et 100°C.
On obtient un borate de l'alcool hydroxybenzylique.
Dans une étape suivante, on libère l'alcool hydroxybenzylique à partir du milieu de condensation, par tout procédé connu, par exemple, saponification, hydrolyse ou alcoolyse.
On préfère mettre en oeuvre un procédé de saponification qui consiste à traiter le milieu réactionnel par une base. La base est de préférence un hydroxyde de métal alcalin et plus préférentiellement, l'hydroxyde de sodium ou de potassium.
La quantité de base introduite varie selon la nature du borate d'aryle. Elle varie entre 2,0 et 4,0 moles, et se situe de préférence aux environs de 2,0 moles, de base alcaline par mole d'acide borique mis en oeuvre.
En fin de saponification, le sel alcalin complexe de l'alcool hydroxybenzylique et de l'acide borique, se retrouve en solution aqueuse qu'il est possible d'utiliser directement lors de l'oxydation conduite en présence de platine et de bismuth, sans qu'il soit nécessaire de séparer les constituants.
Il est donc possible de préparer directement l'aldéhyde salicylique, à partir du phénol, sans isolement des produits intermédiaires formés.
Ainsi, on fait réagir le phénol et l'acide borique (ou l'anhydride borique) pour former un ester borique, puis réaction de ce dernier avec du formaldéhyde permettant d'obtenir un borate de saligénine, qui après saponification à l'aide d'une base alcaline, fournit une solution aqueuse d'un sel alcalin complexe de saligénol et d'acide borique. Il est

ainsi possible d'utiliser cette solution aqueuse directement pour l'oxydation, en présence d'un catalyseur à base de platine et d'un co-catalyseur à base d'un dérivé du bismuth, sans qu'il soit nécessaire de séparer les constituants.

On obtient après oxydation conduite selon l'invention, l'aldéhyde salicylique avec de très bons rendements.

Le procédé de l'invention est donc particulièrement intéressant pour la préparation de l'aldéhyde salicylique qui peut être utilisé, entre autres, pour la préparation de la coumarine.

L'aldéhyde salicylique obtenu selon le procédé de l'invention peut être engagé comme matière première dans la synthèse de la coumarine : celle-ci résultant d'une étape de cyclisation bien connue et largement décrite dans la littérature. On peut citer notamment la préparation de la coumarine selon la réaction de Perkin par réaction de l'aldéhyde salicylique et de l'anhydride acétique, en présence d'acétate de sodium (KIRK-OTHMER - Encyclopedia of Chemical Technology 7, p 198, 3 ème édition).

Les exemples suivants, illustrent l'invention sans toutefois la limiter.

Dans les exemples, l'abréviation RR (rendement) a la signification suivante :

$$RR = \frac{\text{nombre de moles d'aldéhyde formées}}{\text{nombre de moles de saligénol engagées}}$$

Exemples 1 à 4

Les quatre exemples font appel, comme catalyseur à base de métal noble, au platine sous forme d'un catalyseur à 2 % en poids de métal déposé sur du noir de carbone ; la quantité mise en oeuvre, exprimée en poids de platine par rapport à celui de l'alcool à oxyder, est de 0,036 %.

Les exemples sont effectués en présence ou en l'absence d'acide borique.

Lorsque l'acide borique est utilisé, la quantité mise en oeuvre exprimée en poids d'acide borique par rapport à celui de l'alcool à oxyder est de 51 %.

Les exemples sont effectués en présence ou en l'absence d'un co-catalyseur à base de bismuth, en l'occurrence l'oxyde de bismuth ; la quantité mise en oeuvre, exprimée en poids de bismuth par rapport à celui de l'alcool à oxyder est de 0,065 %.

Le protocole opératoire suivi dans tous les exemples, est le suivant :

On dispose d'un ballon de verre de 100 $cm^3$ muni d'un système d'agitation centrale, d'un dispositif de chauffage, d'un thermomètre et qui est relié à une réserve d'oxygène pur, de façon à permettre la lecture du volume de gaz absorbé au cours du temps.

Dans ce réacteur, on charge :

Exemple 1* :

- 8 $cm^3$ d'une solution aqueuse de soude 4N (0,032 mole de soude)
- 72 mg du catalyseur à base de platine (soit 1,44 mg de platine)
- 4 g (0,0323 mole) d'alcool ortho-hydroxybenzylique et 34 $cm^3$ d'eau.

Exemple 2*

On reproduit l'exemple 1 mais on ajoute 2,9 mg d'oxyde de bismuth (2,6 mg de bismuth).

Exemple 3*

On reproduit l'exemple 1, mais on ajoute 8 $cm^3$ d'une solution aqueuse de soude 4N (0,032 mole) et on réduit la quantité d'eau de dilution de 8 $cm^3$ pour conserver la même concentration en alcool dans le mélange à oxyder.

Exemple 4

On reproduit l'exemple 3 mais on ajoute 2,9 mg d'oxyde de bismuth (2,6 mg de bismuth).

Après la charge des réactifs (exemples 1-4), on purge le réacteur à l'oxygène et on le met en relation avec la réserve d'oxygène, en établissant une légère pression correspondant au poids d'une colonne de 30 cm d'eau.

On porte le mélange réactionnel à une température de 45°C, puis l'on met l'agitation en marche (1000 tours/mn).

On maintient le milieu sous agitation à la température précitée et le mélange est ensuite agité à la température

de 45°C jusqu'à l'arrêt de l'oxydation (fin de l'absorption d'oxygène).
Les bilans des réactions, c'est-à-dire les rendements en aldéhyde salicylique par rapport à l'alcool à oxyder, sont déterminés par analyse chromatographique en phase liquide, après acidification du mélange réactionnel.
Les résultats obtenus sont rassemblés dans le tableau ci-après.

Tableau (I)

| Exemple | 1* | 2* | 3* | 4 |
|---|---|---|---|---|
| Acide borique/alcool en poids | 0 | 0 | 0,5 | 0,5 |
| % Pt/alcool en poids | 0,036 | 0,036 | 0,036 | 0,036 |
| Poids de $Bi_2O_3$ | 0 | 2,9 mg | 0 | 2,9 mg |
| Bismuth/platine en poids | 0 | 1,8 | 0 | 1,8 |
| ppm en poids de bismuth/alcool | 0 | 650 | 0 | 650 |
| mole soude/alcool | 1 | 1 | 2 | 2 |
| Durée | 1 h 30 | 45 mn | 8 h | 30 mn |
| Rendement en aldéhyde/alcool engagé (RR) | 77,6 % | 92,2 % | 52 % | 97 % |

* = exemple comparatif

Exemple 5

Dans un ballon tricol de 250 ml muni d'un dispositif d'agitation, d'une gaine thermométrique et d'une colonne équipée d'un rétrogradateur pour opérer à reflux de solvant et séparer l'eau de réaction, on charge :

- 47 g de phénol (0,5 mole),
- 31 g d'acide borique (0,5 mole),
- 15 g de toluène.

On fait distiller pendant 3 heures avec retour de l'entraîneur (toluène) jusqu'à séparation de la quantité théorique d'eau.
Puis, on dilue avec 75 g de toluène, ajoute 16 g de trioxyméthylène en suspension dans 25 g de toluène et maintient à 80°C jusqu'à fin de réaction du formaldéhyde (soit 3 h environ).
La solution toluénique de borate de saligénine obtenue est hydrolysée à température ambiante par une solution de soude préparée en ajoutant 200 g d'eau à 152 g d'une solution de soude à 30 % poids, puis on laisse décanter, et on sépare la solution aqueuse renfermant les sels de sodium du saligénol et de l'acide borique.
Cette solution aqueuse est alors directement soumise à l'oxydation selon le même processus que dans l'exemple 4, sauf qu'il est inutile d'ajouter de l'acide borique.
On fait passer de l'oxygène sous pression atmosphérique dans la solution aqueuse à laquelle on a ajouté :

- 0,8 g de noir à 2 % de platine (16 mg de platine)
- 0,035 g d'oxyde de bismuth (31 mg de bismuth)

jusqu'à ce que le volume d'oxygène absorbé corresponde à la quantité théoriquement nécessaire pour transformer le saligénol en aldéhyde salicylique, ce qui demande environ 1 heure.
On sépare ensuite le catalyseur de la masse réactionnelle, puis on libère l'aldéhyde salicylique formé de son sel de sodium par addition de 200 ml d'acide sulfurique 5N, puis on effectue les opérations d'entraînement à la vapeur d'eau ou d'extraction par un solvant approprié pour isoler l'aldéhyde salicylique.
On obtient ainsi 43 g d'aldéhyde salicylique, ce qui représente un rendement de 68 % par rapport au phénol mis en oeuvre.

Exemple 6*

On reproduit l'exemple 5 mais sans ajouter d'oxyde de bismuth dans la solution aqueuse renfermant les sels de sodium du saligénol et de l'acide borique (avant oxydation).

On obtient 23 g d'aldéhyde salicylique, soit un rendement de 37 % par rapport au phénol mis en oeuvre.

Exemple 7

Dans un ballon tricol équipé d'un thermomètre, d'une colonne à distiller, d'un rétrogradateur, d'un réfrigérant, d'un séparateur, on charge :

- de l'aldéhyde salicylique (600 mmol) préparé selon l'exemple 5,
- de l'anhydride acétique (1,90 mmol) en solution dans de l'acide acétique (3,47 g).

On porte à reflux et l'on introduit de l'acétate de sodium (2,1 mmol) en solution dans de l'acide acétique (3,47 g).

On distille l'acide acétique en maintenant un reflux tel que la température de tête de colonne soit voisine de 118°C.

Après 2 heures 50 de réaction, on dose par chromatographie en phase gazeuse, la coumarine dans le bouilleur ce qui permet de déterminer un rendement en coumarine de 82 %.

## Revendications

1. Procédé de préparation d'un hydroxybenzaldéhyde par oxydation de l'alcool hydroxybenzylique correspondant, en phase liquide, à l'aide d'oxygène moléculaire ou un gaz en contenant, en opérant en milieu aqueux renfermant un agent alcalin, en présence d'un catalyseur à base de platine, caractérisé par le fait que l'oxydation est conduite, en présence d'un dérivé du bore et d'un dérivé du bismuth.

2. Procédé selon la revendication 1 caractérisé par le fait que l'alcool hydroxybenzylique répond à la formule (I) suivante :

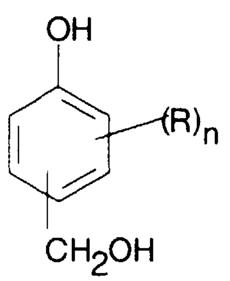

(I)

dans ladite formule, le radical $-CH_2OH$ est en position ortho, méta ou para du groupe hydroxyle et le noyau benzénique peut être éventuellement substitué par un ou plusieurs substituants R, identiques ou différents et n est un nombre inférieur ou égal à 3.

3. Procédé selon la revendication 2 caractérisé par le fait que l'alcool hydroxybenzylique répond à la formule (I) dans laquelle R représente un ou plusieurs substituants qui peuvent être un atome d'halogène, de préférence, fluor, chlore ou brome ou un radical alkyle ou alkoxy ayant de préférence de 1 à 12 atomes de carbone, de préférence de 1 à 4 atomes de carbone.

4. Procédé selon la revendication 1 caractérisé par le fait que l'alcool hydroxybenzylique est le saligénol.

5. Procédé selon l'une des revendications 1 à 4 caractérisé par le fait que le dérivé du bore est choisi parmi les acides boriques, de préférence l'acide orthoborique (ou son précurseur $B_2O_3$), l'acide métaborique, l'acide pyro- ou tétraborique ; les borates métalliques, notamment de métaux alcalins, alcalino-terreux ou d'ammonium sous forme

anhydre ou hydratée, en particulier aux tiers borates, hémiborates, monoborates, diborates, triborates, tétraborates, pentaborates de métaux, de préférence alcalins ou d'ammonium ; les sels doubles contenant du bore, notamment les fluoborates métalliques.

**6.** Procédé selon la revendication 5 caractérisé par le fait que le dérivé du bore est l'acide orthoborique ou l'anhydride borique.

**7.** Procédé selon l'une des revendications 5 et 6 caractérisé par le fait que le rapport entre le nombre de moles de dérivé du bore et le nombre de moles d'alcool hydroxybenzylique varie entre 0,1 et 3,0, de préférence entre 0,9 et 1,1.

**8.** Procédé selon l'une des revendications 1 à 7 caractérisé par le fait que le co-catalyseur est un dérivé organique ou inorganique du bismuth pris dans le groupe formé par: les oxydes de bismuth ; les hydroxydes de bismuth ; les sels de bismuth ou de bismuthyle d'hydracides minéraux, de préférence les chlorure, bromure, iodure, sulfure, sélénure, tellure ; les sels de bismuth ou de bismuthyle d'oxyacides minéraux, de préférence les sulfite, sulfate, nitrite, nitrate, phosphite, phosphate, pyrophosphate, carbonate, perchlorate, antimoniate, arséniate, sélénite, séléniate ; les sels de bismuth ou de bismuthyle d'acides organiques aliphatiques ou aromatiques, de préférence les acétate, propionate, salicylate, benzoate, oxalate, tartrate, lactate, citrate ; les phénates de bismuth ou de bismuthyle, de préférence les gallate et pyrogallate.

**9.** Procédé selon la revendication 8 caractérisé par le fait que le dérivé du bismuth est pris dans le groupe formé par : les oxydes de bismuth $Bi_2O_3$ et $Bi_2O_4$ ; l'hydroxyde de bismuth $Bi(OH)_3$ ; le chlorure de bismuth $BiCl_3$ ; le bromure de bismuth $BiBr_3$ ; l'iodure de bismuth $BiI_3$ ; le sulfate neutre de bismuth $Bi_2(SO_4)_3$ ; le nitrate neutre de bismuth $Bi(NO_3)_3$, $5H_2O$ ; le nitrate de bismuthyle $BiO(NO_3)$ ; le carbonate de bismuthyle $(BiO)_2CO_3$, 0,5 $H_2O$ ; l'acétate de bismuth $Bi(C_2H_3O_2)_3$ ; le salicylate de bismuthyle $C_6H_4CO_2(BiO)OH$.

**10.** Procédé selon l'une des revendications 8 et 9 caractérisé par le fait que la quantité de co-catalyseur utilisée est choisie de manière qu'elle apporte dans la milieu : d'une part, au moins 0,1 % en poids de bismuth métallique par rapport au poids du platine engagé, et d'autre part de 10 à 900 ppm en poids de bismuth métallique par rapport à l'alcool hydroxybenzylique.

**11.** Procédé selon l'une des revendications 1 à 10 caractérisé par le fait que le catalyseur au platine est apporté sous forme de noir de platine, d'oxyde de platine, ou de métal noble lui-même déposé sur des supports divers tels que le noir de carbone, le carbonate de calcium, les alumines et silices activées ou des matériaux équivalents, de préférence le noir de carbone.

**12.** Procédé selon la revendication 11 caractérisé par le fait que la quantité de catalyseur à mettre en oeuvre, exprimée en poids de platine métallique par rapport à celui de l'alcool à oxyder, peut varier de 0,01 à 4 % et, de préférence, de 0,04 à 2 %.

**13.** Procédé selon l'une des revendications 1 à 12 caractérisé par le fait que la réaction d'oxydation est opérée au sein d'un milieu aqueux renfermant de 0,5 à 3 moles d'hydroxyde de sodium ou de potassium par rapport à l'alcool hydroxybenzylique à oxyder.

**14.** Procédé selon l'une des revendications 1 à 13 caractérisé par le fait que la réaction d'oxydation est conduite à une température comprise entre 10°C et 100°C, de préférence entre 20°C et 60°C.

**15.** Procédé selon l'une des revendications 1 à 14 caractérisé par le fait que le dérivé du bore est introduit lors de l'oxydation, avec le catalyseur à base de platine et le co-catalyseur à base de bismuth.

**16.** Procédé selon l'une des revendications 1 à 14 caractérisé par le fait que le dérivé du bore est introduit lors de la préparation de l'alcool hydroxybenzylique.

**17.** Procédé selon l'une des revendications 1 à 14 et 16 caractérisé par le fait que le dérivé du bore contenu dans la solution aqueuse soumise à l'oxydation résulte de la réaction d'un phénol et de l'acide borique (ou l'anhydride borique) pour former un ester borique, ensuite réaction de ce dernier avec le formaldéhyde ou un générateur de formaldéhyde, puis saponification du borate d'alcool hydroxybenzylique obtenu, à l'aide d'une base alcaline conduisant à un sel complexe d'alcool hydroxybenzylique et d'acide borique.

**18.** Procédé de préparation de l'aldéhyde salicylique selon l'une des revendications 1 à 14, 16 et 17 caractérisé par le fait que l'on fait réagir le phénol et l'acide borique (ou l'anhydride borique) pour former un ester borique qui est mis en réaction avec le formaldéhyde ou un générateur de formaldéhyde, puis à saponifier le borate de saligénine obtenu, à l'aide d'une base alcaline conduisant à une solution aqueuse d'un sel complexe de saligénol et d'acide borique qui est soumise à l'oxydation après addition du catalyseur à base de platine et du co-catalyseur à base de bismuth.

**19.** Procédé selon la revendication 18 caractérisé par le fait que le rapport molaire phénol/acide borique engagé est compris entre 0,8 et 3,0, de préférence entre 1,0 et 1,5.

**Patentansprüche**

**1.** Verfahren zur Herstellung eines Hydroxybenzaldehyds durch Oxidation eines entsprechenden Hydroxybenzylalkohols in flüssiger Phase mit Hilfe von molekularem Sauerstoff oder einem diesen enthaltenden Gas, wobei in Gegenwart eines Katalysators auf Platinbasis in einem wäßrigen Medium gearbeitet wird, das ein alkalisches Mittel enthält, dadurch gekennzeichnet, daß die Oxidation in Gegenwart einer Bor- und einer Bismutverbindung ausgeführt wird.

**2.** Verfahren nach Anspruch 1, dadurch gekennzeichnet, daß der Hydroxybenzylalkohol der folgenden Formel (I) entspricht:

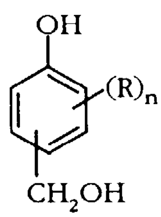

(I)

wobei in der genannten Formel der $CH_2OH$-Rest in ortho-, meta- oder para-Stellung zur Hydroxygruppe steht und der Benzolring gegebenenfalls durch einen oder mehrere gleiche oder verschiedene Reste R substituiert sein kann und n eine Zahl kleiner oder gleich 3 ist.

**3.** Verfahren nach Anspruch 2, dadurch gekennzeichnet, daß der Hydroxybenzylalkohol der Formel (I) entspricht, in der R einen oder mehrere Substituenten bezeichnet, die ein Halogenatom, vorzugsweise Fluor, Chlor oder Brom oder ein Alkyl- oder Alkoxyrest mit 1 bis 12 Kohlenstoffatomen, vorzugsweise mit 1 bis 4 Kohlenstoffatomen, sein können.

**4.** Verfahren nach Anspruch 1, dadurch gekennzeichnet, daß der Hydroxybenzylalkohol Salicylalkohol ist.

**5.** Verfahren nach einem der Ansprüche 1 bis 4, dadurch gekennzeichnet, daß die Borverbindung gewählt ist aus den Borsäuren, vorzugsweise der Orthoborsäure (oder ihrem Ausgangsstoff $B_2O_3$), der Metaborsäure, der Pyro- oder Tetraborsäure; den Metallboraten, insbesondere den Alkali-, Erdalkali- oder Ammoniummetallen in wasserfreier oder hydratisierter Form, insbesondere den Orthoboraten, Hemiboraten, Monoboraten, Diboraten, Triboraten, Tetraboraten, Pentaboraten der Metalle, vorzugsweise der Alkali- oder Ammoniummetalle; den Bor enthaltenden Doppelsalzen, insbesondere aus den Metallfluoroboraten.

**6.** Verfahren nach Anspruch 5, dadurch gekennzeichnet, daß die Borverbindung Orthoborsäure oder Bortrioxid ist.

**7.** Verfahren nach Anspruch 5 oder 6, dadurch gekennzeichnet, daß das Verhältnis zwischen der Anzahl der Mole der Borverbindung und der Anzahl der Mole des Hydroxybenzylalkohols zwischen 0,1 und 3,0, vorzugsweise zwischen 0,9 und 1,1, schwankt.

**8.** Verfahren nach einem der Ansprüche 1 bis 7, dadurch gekennzeichnet, daß der Cokatalysator eine organische oder anorganische Bismutverbindung aus der folgenden Gruppe ist: Bismutoxide; Bismuthydroxide; Bismut- oder Bismutoxidsalze der mineralischen Wasserstoffsäuren, vorzugsweise der Chloride, Bromide, Iodide, Sulfide, Selenide, Telluride; Bismut- oder Bismutoxidsalze der mineralischen Oxosäuren, vorzugsweise der Sulfite, Sulfate, Nitrite, Nitrate, Phosphite, Phosphate, Pyrophosphate, Carbonate, Perchlorate, Antimonate, Arsenate, Selenite, Selenate; Bismut- oder Bismutoxidsalze der aliphatischen oder aromatischen organischen Säuren, vorzugsweise der Acetate, Propionate, Salicylate, Benzoate, Oxalate, Tartrate, Lactate, Citrate; Bismut- oder Bismutoxidphenolate, vorzugsweise Gallussäureester und Pyrogallolester.

**9.** Verfahren nach Anspruch 8, dadurch gekennzeichnet, daß die Bismutverbindung zu der folgenden Gruppe gehört: Bismutoxide $Bi_2O_3$ und $Bi_2O_4$; Bismuthydroxid $Bi(OH)_3$; Bismutchlorid $BiCl_3$; Bismutbromid $BiBr_3$; Bismutiodid $BiI_3$; neutrales Bismutsulfat $Bi_2(SO_4)_3$; neutrales Bismutnitrat $Bi(NO_3)_3$, 5 $H_2O$; basisches Bismutnitrat $BiO(NO_3)$; basisches Bismutcarbonat $(BiO)_2CO_3$, 0,5$H_2O$; Bismutacetat $Bi(C_2H_3O_2)_3$; basisches Bismutsalicylat $C_6H_4CO_2(BiO)OH$.

**10.** Verfahren nach Anspruch 8 oder 9, dadurch gekennzeichnet, daß die Menge des verwendeten Cokatalysators derart gewählt ist, daß sie in das Medium einerseits mindestens 0,1 Gew.-% Bismutmetall, bezogen auf das Gewicht des beteiligten Platins, und andererseits 10 bis 900 ppm (gewichtsbezogen) des Bismutmetalls, bezogen auf den Hydroxybenzylalkohol, einbringt.

**11.** Verfahren nach einem der Ansprüche 1 bis 10, dadurch gekennzeichnet, daß der Katalysator auf Platinbasis in Form von Platinschwarz, Platinoxid oder des Edelmetalls selbst eingebracht wird, das auf verschiedene Träger, z.B. Ruß, Calciumcarbonat, aktive Aluminiumoxide und Kieselsäuren oder äquivalente Materialien, vorzugsweise Ruß, aufgebracht ist.

**12.** Verfahren nach Anspruch 11, dadurch gekennzeichnet, daß die Menge des eingesetzten Katalysators, definiert als das Gewicht des metallischen Platins bezogen auf das des zu oxidierenden Alkohols, zwischen 0,01 und 4 %, vorzugsweise zwischen 0,04 und 2 %, schwanken kann.

**13.** Verfahren nach einem der Ansprüche 1 bis 12, dadurch gekennzeichnet, daß die Oxidation in einem wäßrigen Medium stattfindet, das 0,5 bis 3 mol Natrium- oder Kaliumhydroxid, bezogen auf den zu oxidierenden Hydroxybenzylalkohol, enthält.

**14.** Verfahren nach einem der Ansprüche 1 bis 13, dadurch gekennzeichnet, daß die Oxidation bei einer Temperatur zwischen 10°C und 100°C, vorzugsweise zwischen 20 °C und 60 °C, stattfindet.

**15.** Verfahren nach einem der Ansprüche 1 bis 14, dadurch gekennzeichnet, daß die Borverbindung während der Oxidation zusammen mit dem Katalysator auf Platinbasis und dem Cokatalysator auf Bismutbasis zugegeben wird.

**16.** Verfahren nach einem der Ansprüche 1 bis 14, dadurch gekennzeichnet, daß die Borverbindung während der Herstellung des Hydroxybenzylalkohols zugegeben wird.

**17.** Verfahren nach einem der Ansprüche 1 bis 14 und 16, dadurch gekennzeichnet, daß die Borverbindung, die in der der Oxidation zu unterziehenden wäßrigen Lösung enthalten ist, aus der Reaktion eines Phenols mit Borsäure (oder Bortrioxid) unter Bildung eines Borsäureesters hervorgeht und sich eine Reaktion des letztgenannten mit Formaldehyd oder einem Formaldehydbildner anschließt, gefolgt von der Verseifung des erhaltenen Borats des Hydrobenzylalkohols mit Hilfe einer alkalischen Base zu einem Komplexsalz des Hydrobenzylalkohols und der Borsäure.

**18.** Verfahren zur Herstellung von Salicylaldehyd nach einem der Ansprüche 1 bis 14, 16 und 17, dadurch gekennzeichnet, daß man das Phenol und die Borsäure (oder das Bortrioxid) miteinander reagieren läßt, um einen Borsäureester zu erhalten, der eine Reaktion mit Formaldehyd oder einem Formaldehydbildner eingeht, gefolgt von der Verseifung des erhaltenen Borats des Salicylalkohols, Mit Hilfe einer alkalischen Base, die eine wäßrige Lösung eines Komplexsalzes des Salicylalkohols und der Borsäure ergibt, die nach Zugabe eines Katalysators auf Basis von Platin und eines Cokatalysators auf Bismutbasis der Oxidation unterzogen wird.

**19.** Verfahren nach Anspruch 18, dadurch gekennzeichnet, daß das Molverhältnis Phenol/beteiligte Borsäure zwischen 0,8 und 3,0, vorzugsweise zwischen 1,0 und 1,5, liegt.

## Claims

1. A process for the preparation of a hydroxybenzaldehyde by oxidation of the corresponding hydroxybenzyl alcohol in the liquid phase, using molecular oxygen or a gas containing molecular oxygen, in an aqueous medium containing an alkali, in the presence of a platinum based catalyst, characterised in that oxidation is carried out in the presence of a boron derivative and a bismuth derivative.

2. A process according to claim 1, characterised in that the hydroxybenzyl alcohol has formula (I):

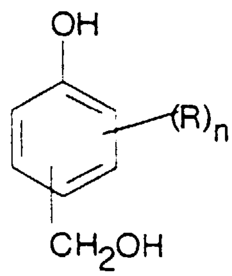

where radical $-CH_2OH$ is in the ortho, meta or para position with respect to the hydroxy group, the benzene nucleus may be substituted by one or more substituents R, which may be identical or different, and n is a number which is less than or equal to 3.

3. A process according to claim 2, characterised in that the hydroxybenzyl alcohol has formula (I) where R represents one or more substituents which may be a halogen atom, preferably fluorine, chlorine or bromine, or an alkyl or alkoxy radical preferably containing 1 to 12 carbon atoms, more preferably 1 to 4 carbon atoms.

4. A process according to claim 1, characterised in that the hydroxybenzyl alcohol is saligenol.

5. A process according to any one of claims 1 to 4, characterised in that the boron derivative is selected from boric acids, preferably orthoboric acid (or its precursor $B_2O_3$), metaboric acid, pyroboric acid and tetraboric acid: metallic borates, in particular those of alkali or alkaline-earth metals or of ammonium in their anhydrous or hydrated forms, in particular metal tertiary borates, hemiborates, monoborates, diborates, triborates, tetraborates or pentaborates, preferably of alkali metals, or of ammonium; or double salts containing boron, in particular metallic fluoborates.

6. A process according to claim 5, characterised in that the oxygenated boron derivative is orthoboric acid or boric anhydride.

7. A process according to claim 5 or claim 6, characterised in that the ratio between the number of moles of oxygenated boron derivative and the number of moles of hydroxybenzyl alcohol is between 0.1 and 3.0, preferably between 0.9 and 1.1.

8. A process according to any one of claims 1 to 7, characterised in that the co-catalyst is an organic or inorganic bismuth derivative selected from the group formed by: bismuth oxides: bismuth hydroxides; bismuth or bismuthyl salts of mineral hydrogen acids, preferably a chloride, bromide, iodide, sulphide, selenide or telluride; bismuth or bismuthyl salts of mineral oxyacids, preferably a sulphite, sulphate, nitrite, nitrate, phosphite, phosphate. pyrophosphate, carbonate, perchlorate, antimonate, arsenate, selenite, or selenate; bismuth or bismuthyl salts of organic aliphatic or aromatic acids, preferably an acetate, propionate. salicylate, benzoate, oxalate, tartrate, lactate or citrate; and bismuth or bismuthyl phenates. preferably the gallate or pyrogallate.

9. A process according to claim 8, characterised in that the bismuth derivative is selected from the group formed by: bismuth oxides $Bi_2O_3$ and $Bi_2O_4$; bismuth hydroxide $Bi(OH)_3$; bismuth chloride $BiCl_3$; bismuth bromide $BiBr_3$; bismuth iodide $BiI_3$; neutral bismuth sulphate $Bi_2(SO_4)_3$; bismuth nitrate $Bi(NO_3)_3,5H_2O$; bismuthyl nitrate $(BiO)NO_3$; bismuthyl carbonate $(BiO)_2CO_3,0.5H_2O$; bismuth acetate $Bi(C_2H_3O_2)_3$; and bismuthyl salicylate $C_6H_4CO_2(BiO)(OH)$.

10. A process according to claim 8 or claim 9, characterised in that the quantity of co-catalyst used is selected such

that it provides in the medium: at least 0.1 % by weight of metallic bismuth with respect to the weight of platinum used, and 10 to 500 ppm by weight of metallic bismuth with respect to the hydroxybenzyl alcohol.

**11.** A process according to any one of claims 1 to 10, characterised in that the platinum catalyst is in the form of platinum black, platinum oxide, or the noble metal itself deposited on a support such as carbon black, calcium carbonate, activated aluminas and silicas, or equivalent materials, preferably carbon black.

**12.** A process according to claim 11, characterised in that the quantity of catalyst used, expressed as the weight of metallic platinum with respect to that of the alcohol to be oxidized, is 0.01 % to 4 %, preferably 0.04 % to 2 %.

**13.** A process according to any one of claims 1 to 12, characterised in that oxidation is carried out in an aqueous medium containing 0.5 to 3 moles of sodium or potassium hydroxide with respect to the hydroxybenzyl alcohol to be oxidized.

**14.** A process according to any one of claims 1 to 13, characterised in that oxidation is carried out at a temperature of between 10°C and 100°C, preferably between 20°C and 60°C.

**15.** A process according to any one of claims 1 to 14, characterised in that the boron derivative is introduced during oxidation, with the platinum based catalyst and the bismuth based co-catalyst.

**16.** A process according to any one of claims 1 to 14, characterized in that the boron derivative is introduced during preparation of the hydroxybenzyl alcohol.

**17.** A process according to any one of claims 1 to 14 and 16, characterised in that the boron derivative contained in the aqueous solution to be oxidized is produced by reacting a phenol with boric acid (or boric anhydride) to form a boric ester, which is then reacted with formaldehyde or a formaldehyde generator, followed by saponification of the hydroxybenzyl alcohol borate obtained using an alkaline base to produce a complex salt of hydroxybenzyl alcohol and boric acid.

**18.** A process for the preparation of salicylic aldehyde according to any one of claims 1 to 14, 16 and 17, characterised in that phenol and boric acid (or boric anhydride) are reacted to form a boric ester which is reacted with formaldehyde or a formaldehyde generator, followed by saponification of the saligenine borate obtained, using an alkaline base, to produce an aqueous solution of a complex salt of saligenol and boric acid which is oxidized after addition of a platinum based catalyst and a bismuth based co-catalyst.

**19.** A process according to claim 18, characterised in that the molar ratio of phenol/boric acid used is between 0.8 and 3.0, preferably between 1.0 and 1.5.